# EUROPEAN PATENT APPLICATION

(11) **EP 3 162 899 A1**
(43) Date of publication of application: **03.05.2017**
(21) Application number: 15192136.8
(22) Date of filing: 29.10.2015
(51) Int. Cl.: C12Q 1/68

(54) **BIOMARKER FOR BREAST CANCER**

(71) Applicant: Rheinisch-Westfälische Technische Hochschule (RWTH) Aachen, 52056 Aachen (DE)
(72) Inventor: Dahl, Edgar, 4721 Kelmis (BE); Kloten, Vera, 50823 Köln (DE); Mijnes, Jolein, 6372HV Landgraaf (NL)
(74) Representative: Kröncke, Rolf

(57) **Abstract**

The present invention relates in a first aspect to new methods for diagnosing or identifying breast cancer or pre-forms thereof in a subject as well as methods for monitoring the development or progress of breast cancer or pre-forms thereof in a subject and methods for the stratification of the therapeutic regimen of a subject suspected to be afflicted with breast cancer or being at risk of developing breast cancer or pre-forms thereof whereby the level or amount of methylation of at least one of the promoters selected from SPAG6-, PER1- and NKX2-6-gene in a sample of said subject is determined followed by the step of diagnosing or identifying breast cancer or pre-forms thereof based on the level on amount of methylation of the at least one promoter. Further, the present invention relates to the use of a kit in a method according to the present invention. Finally, the present invention relates to new biomarkers, namely, the promoter of the SPAG6-gene, the promoter of the PER1-gene and the promoter of the NKX2-6-gene, in particular, the level or amount of methylation of said promoters is a biomarker for breast cancer.

SEQUENCE LISTING

<110>  Rheinisch-Westfaelische Technische Hochschule Aachen (RWTH)

<120>  Biomarker for breast cancer

<130>  4589-010 EP-1

<160>  20

<170>  PatentIn version 3.5

<210>  1
<211>  177
<212>  DNA
<213>  Homo sapiens

<400>  1
tcccttggcg cgtggggctc tcccggcgct tggtcccgac ccgcgccgca gtcaggatac      60

cacggcaatc tggggtcgct ttgtaaggag ctccgacctc cgggcagtgc aggactcgac     120

ggcgcgcgat gtcccggcgg ctccgcgagc ctggcgcgct gagagcccca gcagtcc        177


<210>  2
<211>  540
<212>  DNA
<213>  Homo sapiens

<400>  2
cgatggcagg ttgccggctt cggtccttgt ggtggggaag cagcgcaggg gctaaagaag      60

gaaaggttgt gttgtcttga ccagaatgac tgggatccag gcgagagtca ggttgagggg     120

cttttgcgcc gagggcagtg gccgttgtgc ttcgtcctgg ggaagccagg accaaccctg     180

ggatcctaat aggccgtccc acttcttacg gccaccgagc catcctggga ctggggatat     240

gtaagggatg cgtgaccccc ccggccgcgc ccaccagtgt gagagtgggc cgttccccca     300

cttccgccgg gaaatggggg aggggtcgct cctcccgccc tcctgtggtc cctccagcaa     360

ccgctgagct cagcagctga cgtcggtttc cctggcgacc gtggcggcgg cggaagcgcg     420

tggtggggcc gcgcacgtcg gcgcgcatgt gcagcggggg tggcaccgcc cccggataaa     480

attagcctgg aggcctaaat ataggaggcg accagctcac ctcctgctcc gaggccttgg     540


<210>  3
<211>  420
<212>  DNA
<213>  Homo sapiens

<400>  3
gaagttcagg gtgccgctcc tcacccggac ctcctcggcc ccgccccatc cgccttcggg      60

atgctgctga gccccgtcac ctccaccccc ttctcggtca aggacatcct gcgactggag     120

cgcgagcgga gctgccccgc ggcttcgcca catccgcggg tgcggaagag cccggaaaac     180

tttcagtacc tgagaatgga cgcagagccg cgagggtcag aggttcacaa cgctggtggc     240

ggcggcggtg acagaaagct ggatggttcg gagcctcctg ggggtccctg tgaggcagtc 300

ttggagatgg acgcggaacg gatgggggag ccacgtgagt aagcgctcag gtcttgcctc 360

ctacgggggg aatgtggggg cgggggcgtg atccatgggt gcatggggaa aatacgcctc 420

<210> 4
<211> 3375
<212> DNA
<213> Homo sapiens

<400> 4
ctccagttcg gcattagttt cttcctaatg tccactctgc ccgaagtgac aagcgggggc 60

atgtggagac tcagctccag gttcctggac gggctcagcc acccccagaa agctaatgaa 120

tgctcaacca gggcttccag atgcccaggg gacagagcag gagatgccgg ggaatggggc 180

tttccttgca gttcaggagg gccctgcccc aggcccagaa gtagaaggga aagcggctgt 240

tttggcggta aacagtaatg tggggagtgc tgcagagaaa ggcagtcttg gggtttcaag 300

ctggagagca gtcagctaca ctcaggacct ctggccatcc ctgccttcac ctgctgtttg 360

gcctgatcgt ctaacttctc tgattctcca ctacccactc cttattacgt ttttgagact 420

tgtcaaagtt ttatattagg gctaactggg acgcatacaa atctggtaac ttcgccaggg 480

cgggaagtta ggaaggagca gagctggctg caggtgtctg gtcctgacca ctcctctatg 540

ccacccttga ggagcttgct gactttctca tgacgttctc ccattccagg agctgcaagt 600

gcgttatcct ggctggagca cggtgtcaat cacggcagac taaggccagc ggtgatggct 660

tgaatgccag gctgggggct gggatttttc ctgaggattt cacaggacag aggttggctt 720

ggaaagacca aggtgggact gaggaacatt ccccctaccc ccaacctcgg tgggctgttg 780

caagcctgga ggccagagaa gacgggcctg ggatgccgcg ggcgcagggg caggcagtga 840

aggagatggc tgccttcggt agagctggtc gctgaggcag aagaggaggg cgtggggcgt 900

ggggcgtgag gtggccggcg ccccggctgg ccaatggccg ggctgcggcc cctccgcggg 960

gcggggtggg cctggtgggc gggcggggct cggggcgggg gcggagaggg agcctggtgg 1020

gcgggcgggg cgcgtcttgc gggctccctc gggtaccggc gctgccgcac cccgccgcgc 1080

tcccgcaccc gcggcccgcc caccgcgccg ctcccgcatc tgcacccgca gcccggcggc 1140

ctcccggcgg gagcgagcag atccagtccg gcccgcagcg caactcggtc cagtcggggt 1200

gggtgagggg cggcggcggg ggaggggacg actctgctga gctcagcctc tcttggtgga 1260

tgtggggcgg ggcgctcgag taggacccga cgccaagggg agggggctga cctgtgcttg 1320

gtccacccca ggtaggggct gagagaggct tgaggtggaa gtgggggtcg ggcactctga 1380

cctggtcgag gaggggctag ggtttgaacc ggggacagag tctaggtgag ctggggcttg 1440

ggagctatta gcgtagagga tccgggttcg gttgctctgg cgagggctcc agcatcacag 1500

gtgaggagca gagctcagct tgtgctcgag gatgggggtg cggggtcctg gggcgatcgg 1560

ggctcgggct ggagcgggtg gggtcgcgtc ttttcgggtg taggggagct gcgttttcag 1620

gggcctcggt ttcctcccgt ctccgggcgg gtgcgggaaa gaggggcgcc aggggcgggc 1680

ggcggggcgg aacggggcgg ggcggcgggc ccagcgaggg gggtggtccg cgcgcctctg 1740

atcgcgttcc gggacacaca ggcggcggct gcgggcgcag agcggagatg cagcggcttg 1800

gggccaccct gctgtgcctg ctgctggcgg cggcggtccc cacggccccc gcgcccgctc 1860

cgacggcgac ctcggctcca gtcaagcccg gcccggctct cagctacccg caggaggagg 1920

ccaccctcaa tgagatgttc cgcgaggttg aggaactgat ggaggacacg cagcacaaat 1980

tgcgcagcgc ggtggaagag gtgggtgcgc cctggcggcg cggggctctg agaagcgcca 2040

gactgggggc agcgaagtct gggaaggcat ggaagagcgc tggccaggga cctcgggtcc 2100

cgcgcagggc ctgggaaggg ccgacagcag atgcggagtg acctggaggc agcgcacggt 2160

gatccctact cccgcctcgt tttgcctcct tgccaaaaag tctggatctg agcctgtcca 2220

ccgagaggct ctgcactgga ccctccctag gtagctgggg ccaccacctg cctgcagttg 2280

gcgtcaggca tgccgttggc cttccctatt gtcagcagag gtttgcaggg cccctggtat 2340

tccagcactg tggtttcaca gaaacccggg acaagctcca ggcatcgcca agttctagct 2400

tgtccgttat gctttgagct tgttaggatg ccatgcaggg acaaagaggc aaagagccct 2460

gagctttgct ggcagtctgt ctgggctgtc ttgcttctca cgcaatgtgg gggcagagag 2520

ggaagatgga aatcctaaat ctcagcactg tatgagtggc ggatgaggat gtgagaggca 2580

ataccccctc cacccaacgg tgcttcgcag ttgtcctggg ctcaggtcct cctctgccac 2640

tcagtagcag ggtgacattg ggcaaatcac ttaatatcct gcacctcagt ttcctgtgaa 2700

aactaaatgt catatgtgtg ggcacctggc aaataatagg tgcgcagtat ttagcaacta 2760

ttatcagata ccacgtacct tagcaaagct ttgcctagct tctgccctcc atcacctcca 2820

ggtctaaaca ctgtgggtgg catcttgttc tgtcctctgt gatggtcacc cacgcatgcc 2880

cagggctacc tgggcaggag atgtcactgg cccagccctc tctgtccagg tgcggggagc 2940

tgtccctggt gcctggggtg ggtagacgag ggcgctgttg ggcccaatct gtctgtagcc 3000

tgtgtctata gagaggagcc agccgtccta gtgaaggagg cagcaggaaa ccaatgtcca 3060

ggctggttgg cccctggtct ggcttcacat ccctgctgtc tccagcttgg tatccccctt 3120

gggcaaagcc ttaactgctg gcccttgtct ccagggggaa gttgctttct ccccgcagcc 3180

ctttgtagtt tggggctatt tcccctccca ctccacaaca gttagcctgt ggagtttgca 3240

gtgaagggga cactggcttg ggaggcagtg aaatgggcag ctgtccttga atatccactg 3300

gacaggccca gatcccaaga cccatgatct ttctgtttcc tgtgattgca aaacaatcat 3360

gacatggagc aatta                                                    3375

<210>  5
<211>  1680
<212>  DNA
<213>  Homo sapiens

<400>  5
tggtccttaa agggtctgtc actcacctca cctatttaga tgaaattatc acagagacat      60

aaaaaacatc ctttgcaaca tgaaataaac aagtagcccc aactgcaatg tgtatcacca     120

gataaaaaag acgcactcag cctgtagaag gccaggtgtg gacggctggt gggtaagaat     180

tcttccaatg gcaggatgga agtatcacca atagtttgta actaagatac atactggcac     240

tcacttacat gattaataac cagaaactca gaaatactac acgctaccaa gatacttccc     300

tcgtctcctc tgtttcctta gaacataaag gctggcagcc ttgctagtcc ctcctgcgct     360

agactgcagg tagtatcctt tgtcctgcct attaagcgtc cctggccaat gcagagggga     420

cgatctgccg gcttcaggct ggcaggtgcg gggtgggctg tgtgctgagg accaggctgg     480

ccttctcgcg tcctctggcg acagaaatta agcaagtccg caaagaagca tattgcacct     540

tgagggagag gaagaaagag ttgcgggggc gcgtggggta gggcccggga ggcagtgggt     600

gttggcaaga aacggagtag gaggctctac agaggagact ggtccttggt taagtgtcac     660

agccgcagga tgggagggtg tgggaggggt ccgtggggcc aacacaggtg gcccctgctg     720

tgcgcggcga ggcaggcttg gaatgccttc tccccgaaca gagctagccc agcactgggg     780

gctggaggcg cggctctggt gggactgagg ccgggaagcg gcgccgggcg gggtggggac     840

tggggaggag gcggggctgg tgtgggggcc acagggaggc gggccgggtt taagggggtg     900

gctgtaggtg gggtctggga agttggcctc ggtggggaca gggacaggag aggtggggcc     960

gggactgggg aggtgggggg aaatgggggc gtggagaaac ggagcttggg gttggggacc    1020

tggggaagct gggtcctgac tggggacagg gaggcgaggc cgggcttggg gggtgtccag    1080

ggaggcaggg tgtgggaagg tctggggcgg ggaagtgggg tctgggctgg ggtgggagag    1140

ttggagtcca gaaggggacc gcggggtgga aggggtccgg gctgggaccg gggtaggcgg    1200

agtccaggag gggacgcagg gtgggcgggg tccgggctgg gtccgtgggg tgggcggggt    1260

ccgggctggg acgcggggtg ggcggggtcc gggctgggtc cgtggggtgg gcggggtccg    1320

ggctgggtcc gtggggtggg cggagtccag gaggggaccg cagggtgggc ggggtccggg    1380

ctgggtccgt ggggtgggcg gggtccgggc tgggtccgtg gggtgggcgg ggtccgggct    1440

gggaccgcgg ggtgggcggg gtccgggctg ggtccgtggg gtgggcgggg tccgggctgg    1500

gaccgcgggg tgggcggccc cgagagcgtc ccgcagtggc tggagccctg ggcgctgcaa    1560

agcgtgtccc gccgggtccc cgagcgtccc gcgccctcgc cccgccatgc tcctgctgct    1620

ggggctgtgc ctggggctgt ccctgtgtgt ggggtcgcag gaagaggcgc agagctgggg     1680

<210>  6
<211>  37
<212>  DNA
<213>  artificial sequence

<220>
<223>  primer

<400>  6
taaggagttt ygattttagg gtagtgtagg gatattt                              37

<210>  7
<211>  27
<212>  DNA
<213>  artificial sequence

<220>
<223>  primer

<400>  7
cccraactcr aaaactcaaa ctactaa                                         27

<210>  8
<211>  18
<212>  DNA
<213>  artificial sequence

<220>
<223>  primer

<400>  8
gggtagtgta gggatatt                                                   18

<210>  9
<211>  23
<212>  DNA
<213>  artificial sequence

<220>
<223>  primer

<400>  9
tttagtaggg aaatggggga ggg                                             23

<210>  10
<211>  20
<212>  DNA
<213>  artificial sequence

<220>
<223>  primer

<400>  10
ccacratcrc caaaaaaacc                                                 20

<210>  11
<211>  15
<212>  DNA
<213>  artificial sequence

<220>
<223>  primer

<400>  11
gaaatggggg agggg                                                        15


<210>  12
<211>  35
<212>  DNA
<213>  artificial sequence

<220>
<223>  primer

<400>  12
gggtgyggaa gagttyggaa aatttttagt atttg                                   35


<210>  13
<211>  28
<212>  DNA
<213>  artificial sequence

<220>
<223>  primer

<400>  13
caaaaaactc craaccatcc aactttct                                          28


<210>  14
<211>  19
<212>  DNA
<213>  artificial sequence

<220>
<223>  primer

<400>  14
ggaaaatttt tagtatttg                                                    19


<210>  15
<211>  26
<212>  DNA
<213>  artificial sequence

<220>
<223>  primer

<400>  15
aggggtgggt tgtgtgttga ggatta                                            26


<210>  16
<211>  32
<212>  DNA

<213>  artificial sequence

<220>
<223>  primer

<400>  16
cctactccct ttcttaccaa cacccactac ct                                    32


<210>  17
<211>  18
<212>  DNA
<213>  artificial sequence

<220>
<223>  primer

<400>  17
ggttgtgtgt tgaggatt                                                    18


<210>  18
<211>  28
<212>  DNA
<213>  artificial sequence

<220>
<223>  primer

<400>  18
ttagggaggt agtgggtgtt ggtaagaa                                         28


<210>  19
<211>  32
<212>  DNA
<213>  artificial sequence

<220>
<223>  primer

<400>  19
ctctattcca aaaaaaaaca ttccaaacct ac                                    32


<210>  20
<211>  19
<212>  DNA
<213>  artificial sequence

<220>
<223>  primer

<400>  20
aaaaaaaaac attccaaac                                                   19

## Description

The present invention relates in a first aspect to new methods for diagnosing or identifying breast cancer or pre-forms thereof in a subject as well as methods for monitoring the development or progress of breast cancer or pre-forms thereof in a subject and methods for the stratification of the therapeutic regimen of a subject suspected to be afflicted with breast cancer or being at risk of developing breast cancer or pre-forms thereof whereby the level or amount of methylation of at least one of the promoters is selected from *SPAG6-, PER1-* and *NKX2-6*-gene in a sample of said subject determined followed by the step of diagnosing or identifying breast cancer or pre-forms thereof based on the level on amount of methylation of the at least one promoter. Further, the present invention relates to the use of a kit in a method according to the present invention. Finally, the present invention relates to new biomarkers, namely, the promoter of the *SPAG6*-gene, the promoter of the *PER1*-gene and the promoter of the *NKX2-6-*gene, in particular, the level or amount of methylation of said promoters is a biomarker for breast cancer.

### Background of the invention

Breast cancer remains the most frequently diagnosed cancer and the leading course of cancer death in European women. Worldwide about 1.7 million women are newly diagnosed with breast cancer every year, in Europe approximately 450.000 women will be diagnosed yearly with breast cancer comprising approximately 28% of all cancers in female patients.

15% of cancer related deaths in women is due to breast cancer. Localised, early stage breast cancer has a favourable prognosis, with a five year survival rate of up to 100%. However, the five year survival deadline declines to about 20 to 22% when the tumour has metastasised.

Although clinical examination using ultrasound provide a preliminary screening method, the most commonly used imaging techniques are mammography and magnetic resonance imaging (MRI). Actually, mammography has become the standard of care in breast cancer screening and the mammograms are useful in that they are reliable and sensitive enough to detect early stages of breast cancer including ductal carcinoma in situ (DCIS).

However, the limitations of mammography are well recognized and include poor accuracy in women with dense breast tissue and, thus, a relatively high rate of false positive cases. In addition, women disregard this method as being uncomfortable and the compliance rate is about 50 to 70% only. Problematically, clinically occult early stage breast cancer and pre-cancerous tissue is often similar in appearance to a benign breast lesion, resulting in unnecessary subsequent biopsies. Furthermore, indolent tumours (i.e. benign tumours that would never have killed the patient) are falsely identified. Finally, it has been recognized that mammography provides no breast cancer survival benefit in the elderly woman (age 70+).

Further, the sensitivity of mammography is typically in the range of 70% up to 90%. Thus, the remaining cases of breast tumours are missed by mammography.

MRI requires no breast compression and offers excellent imaging around dense breast tissue, the high sensitivity of magnetic resonance imaging (MRI) often results in false positives as well, which call for unnecessary follow-up examination and invasive biopsies.

Hence, screening tests are desirable that could complement mammography or MRI, or even stand alone as a primary screening tool. For example, screening tests based on liquid biopsies, namely, using body fluids, are desired.

In this connection, the determination of promoter methylation of tumour suppressing genes in DNA of easily accessible body fluids, like serum or plasma, is a rapidly growing research field in cancer detection. Basically, the method relies on detecting methylation in circulating cell free DNA (cfDNA) present in the bodily fluid, e.g. in the bloodstream through cellular necrosis, apoptosis, or spontaneous release of DNA into circulation from primary and metastatic tumours.

The diagnostic potential of various genes, like *RASSF1A* and *APC* promoter methylation as well as *ITIH5* or *DKK3* has been investigated in various studies. For example, Kloten V., et al, Breast Cancer Research, 2013, 15:R4 describe promoter hypermethylation of the tumour suppressor genes *ITIH5, DKK3* and *RASSF1A* as novel biomarkers of blood based breast cancer screening.

Of note, the *ITIH5* gene has been described also as a marker for bladder cancer, namely, WO 2013/144362 A1 describes a method for diagnosing or identifying bladder cancer in a subject comprising determination the level or amount of methylation of the promoter of *ECRG4-* and/or *ITIH5*-gene in a sample of said subject and diagnosing or identifying bladder cancer based on the level or amount of the methylation of a promoter of the *ECRG4-* and/or *ITIH5-*gene.

That is, supposed specific biomarkers are reduced in their overall specificity when analysing additional serum samples from non-breast tumour patients. In addition, fibroadenoma tumours, the most common benign tumours in women, typically found during the middle and later reproductive years, are a potential source for hypermethylated cfDNA, indicating that high specificity in benign disease controls is mandatory to establish a suitable biomarker panel for early breast cancer detection.

DNA hypermethylation as a biomarker would be a reasonable tool for stand-alone screening or for combined screening for various types of cancer including breast cancer. The samples are easily usable and workable. In addition, hypermethylation is very frequent and represents an early event in breast cancer developments, thus, allowing to identify and determine breast cancer and pre-forms thereof at a very early stage.

In addition, due to the use of bodily fluids, i.e. liquid samples, this method may be automated and allows to reduce the costs accordingly.

An object of the present invention is to provide methods allowing for diagnosing or identifying breast cancer and pre-forms thereof in a subject suffering or being at risk of developing breast cancer or pre-forms thereof based on a minimal set of genes with high specificity and high sensitivity. Another object of the present invention is to provide a method for monitoring the development or progress of breast cancer or pre-forms in a subject suspected to be afflicted or having a risk of developing breast cancer. Further, another aim of the present invention is to provide methods for the stratification of the therapeutic regimen of a subject suspected to be afflicted with breast cancer or being at risk of developing breast cancer or pre-forms thereof which should substitute or may be used in combination with mammography breast cancer detection.

The present invention aims for providing new methods as well as biomarkers and kits including the same, particularly useful in the issues described above.

### Summary of the present invention

In a first aspect, the present invention relates to a method for, preferably early, diagnosis of breast cancer or pre-forms in a subject, comprising a) determining the level or amount of methylation of the promoter of the *SPAG6-*and/or *PER1-* and/or *NKX2*-*6*-gene, in particular, of the nucleic acid sequences of SEQ ID No. 1 and/or SEQ ID No. 2 and/or SEQ ID No. 3, in a sample of said subject; and b) diagnosing or identifying breast cancer or pre-forms thereof based on the level or amount of methylation of the promoter of the *SPAG6-* and/or *PER1-* and/or *NKX2-6*-gene.

DNA methylation is the main epigenetic modification in humans. It is a chemical modification of DNA performed by enzymes called DNA methyltransferases, in which a methyl group (m) is added to specific cytosine (C) residues in DNA. In mammals, methylation occurs only at cytosine residues adjacent to a guanosine residue, i.e. at the sequence CG, also called the CpG dinucleotide. In normal cells, methylation occurs predominantly in regions of DNA that have few CG base repeats, while so called CpG islands, regions of DNA that have long repeats of CG bases, remain non-methylated. Gene promoter regions that control gene transcription and thus protein expression are often CpG island-rich. Aberrant methylation of these normally non-methylated CpG islands in the promoter region causes transcriptional inactivation or silencing of important functional genes in human cancers, i.e. tumor suppressor genes.

In a further aspect, the present invention relates to a method for monitoring the development or progress of breast cancer or pre-forms thereof in a subject afflicted with, suspected to be afflicted with or having a risk of developing breast cancer, comprising
a) determining the level or amount of methylation of the promoter of the *SPAG6-* and/or *PER1-* and/or *NKX2-6*-gene, in particular, of the nucleic acid sequences of SEQ ID No. 1 and/or SEQ ID No. 2 and/or SEQ ID No. 3, at a first time point and, optionally,
b) determining the level or amount of methylation of the promoter of the *SPAG6-* and/or *PER1-* and/or *NKX2-6*-gene, in particular, of the nucleic acid sequences of SEQ ID No. 1 and/or SEQ ID No. 2 and/or SEQ ID No. 3, at a second time point; and optionally,
c) comparing the level or amount of methylation determined in step a) to the level or amount detected in step b) or to a reference value.

Another embodiment of the present invention relates to a method for the stratification of the therapeutic regimen of a subject suspected to be afflicted with breast cancer or being at risk of developing breast cancer or pre-forms, optionally in combination with mammography-based breast cancer detection and/or MRI-based breast cancer detection, comprising
a) determining the level or amount of methylation of the promoter of the *SPAG6-* and/or *PER1-* and/or *NKX2-6*-gene, in particular, of the nucleic acid sequences of SEQ ID No. 1 and/or SEQ ID No. 2 and/or SEQ ID No. 3; and
b) determining the therapeutic regimen of said subject based on the level or amount of methylation of the promoter of *SPAG6-* and/or *PER1-* and/or *NKX2-6-*gene, optionally further on the basis of the results of mammography-based breast cancer recognition and/or MRI-based breast cancer detection.

That is, the present inventors recognized that the level or amount of methylation of the promoter of the *SPAG6-* and/or *PER-1-* and/or *NKX2*-gene in a sample of a subject, in particular, a bodily fluid sample including serum and plasma, represents a suitable biomarker for diagnosing or identifying breast cancer or pre-forms thereof as well as for monitoring the development of progress of breast cancer or pre-forms thereof in a subject, e.g. relapse of the tumor after treatment or the treatment success of a regimen and, in addition, for the stratification of the therapeutic regimen of a subject suspected to be afflicted with breast cancer or being at risk of developing breast cancer or pre-forms thereof.

Moreover, the present invention relates to a biomarker for breast cancer or pre-forms thereof which is at least one of the promoters of *SPAG6-* and/or *PER1-* and/or *NKX2-6*-gene, in particular, the level or amount of methylation of said promoter of the respective genes.

Finally, the present invention provides a test kit for use in a method according to the present invention comprising means for determining the level or amount of methylation of the promoter of the *SPAG6-* and/or *PER1-* and/or *NKX2-6*-gene in a bodily fluid of a subject to be tested, in particular, of a serum sample or plasma sample, and instructions how to use said test kit for a method according to the present invention.

It is preferred that said test kit is a test kit allowing pyrosequencing or qMSP (quantitative methylation-specific PCR), in particular, it is preferred that the method as well as the test kit is effected or allow pyrosequencing.

### Brief description of the drawings

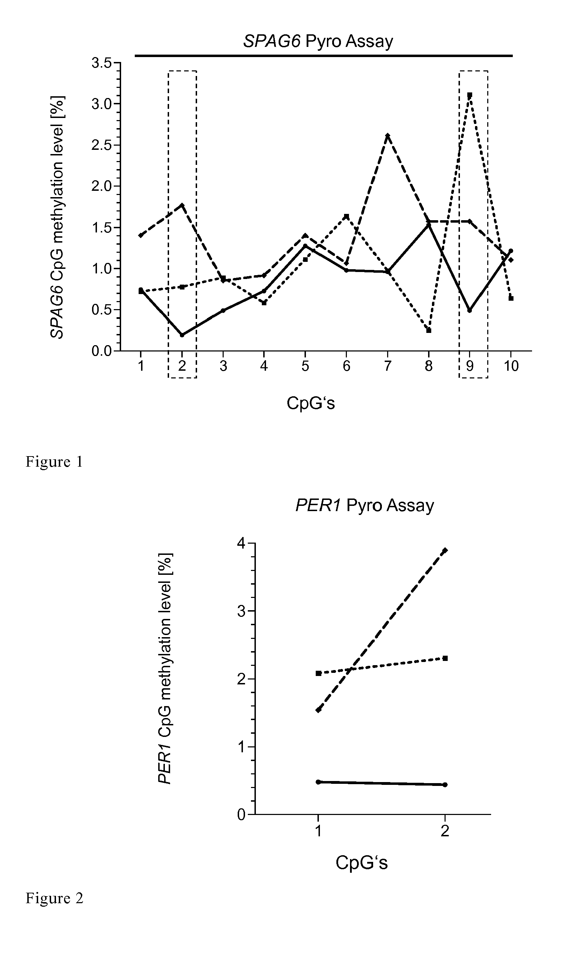

Figure 1: **Frequency of *SPAG6* methylation determined by pyrosequencing.** Mean methylation rates for each CpG site of DNA preparations derived from serum samples of breast cancer patients (DCIS and early tumor) and healthy donors, respectively. Arrows indicate CpG sites with highest mean difference in methylation rate between tumour serum samples and healthy serum samples (control group).
Figure 2: **Frequency of *PER1* methylation determined by pyrosequencing.** Mean methylation rates for each CpG site of DNA preparations derived from serum samples of breast cancer patients (DCIS and early tumor) and healthy donors, respectively. Arrows indicate CpG sites with highest mean difference in methylation rate between tumour serum samples and healthy serum samples (control group).

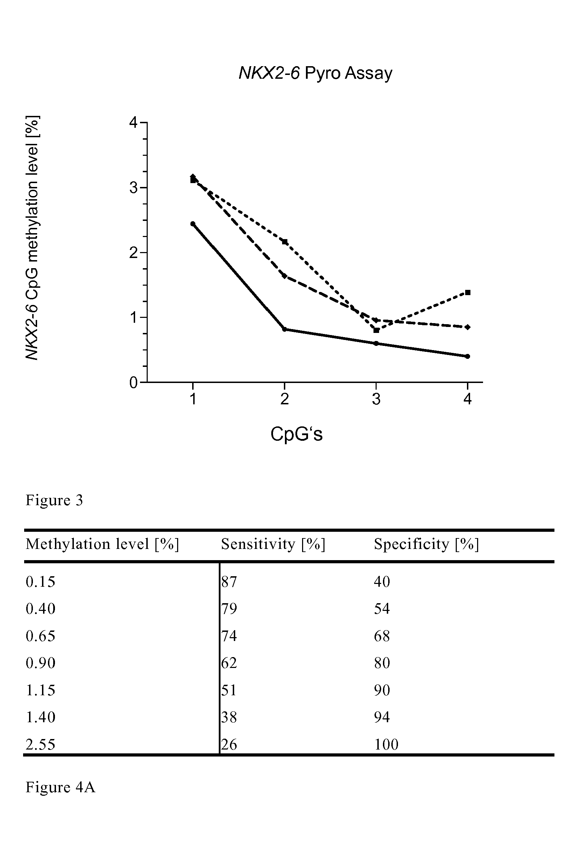

Figure 3: **Frequency of *NKX2-6* methylation determined by pyrosequencing.** Mean methylation rates for each CpG site of DNA preparations derived from serum samples of breast cancer patients (DCIS and early tumor) and healthy donors, respectively. Arrows indicate CpG sites with highest mean difference in methylation rate between tumour serum samples and healthy serum samples (control group).

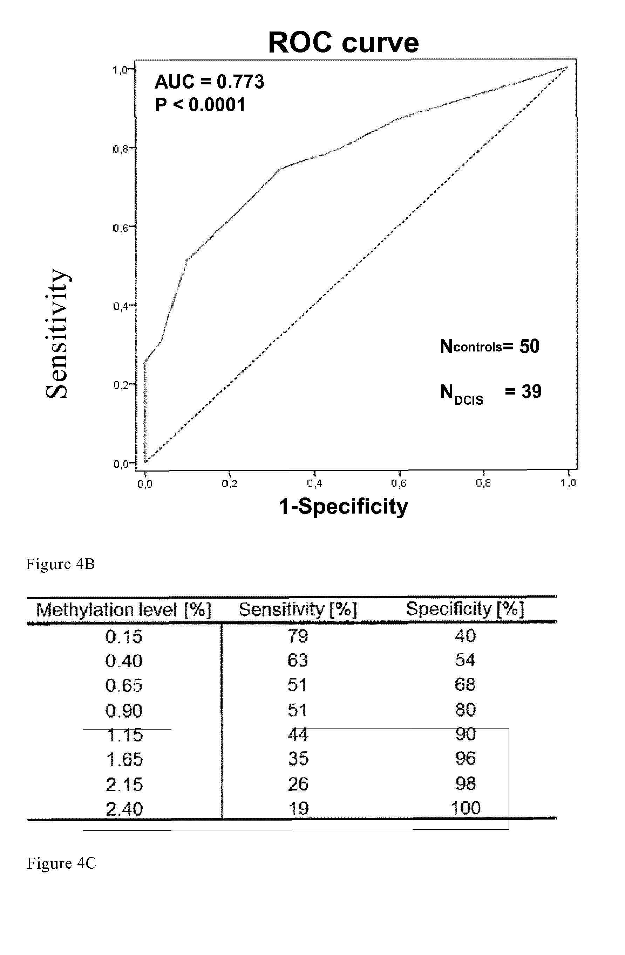

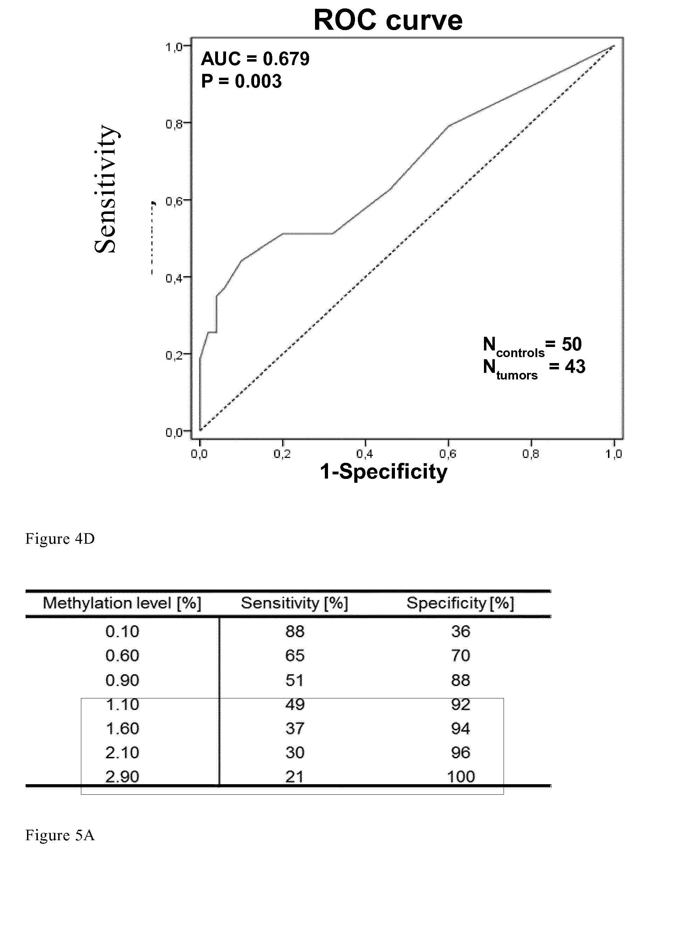

Figure 4: **A)** Table illustrating the mean methylation values of a two panel locus (*SPAG6* and *NKX2-6)* of breast cancer (non-invasive DCIS) associated serum samples compared to control samples. As shown the methylation level allows to determine sensitivity and specificity of the analysis **B)** ROC curve analysis of the two panel biomarker performance is shown. A cut-off value of 1.4% methylation was defined for positive detection of disease; the specificity of the panel is still 94% with a sensitivity of 38%. **C)** Table illustrating the median methylation values of a two panel locus (*SPAG6* and *NKX2-6)* of breast cancer (invasive tumor) associated serum samples compared to control samples. As shown the methylation level allows to determine sensitivity and specificity of the analysis **D)** ROC curve analysis of the two panel biomarker performance is shown. A cut-off value of 1.6% methylation was defined for positive detection of disease; the specificity of the panel is still 94% with a sensitivity of 35%.

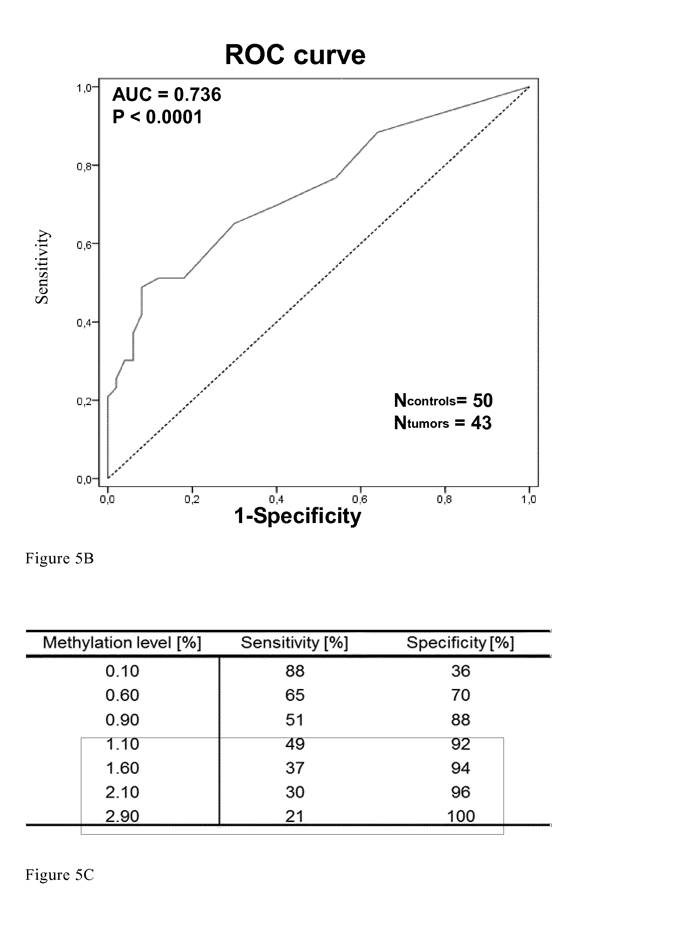

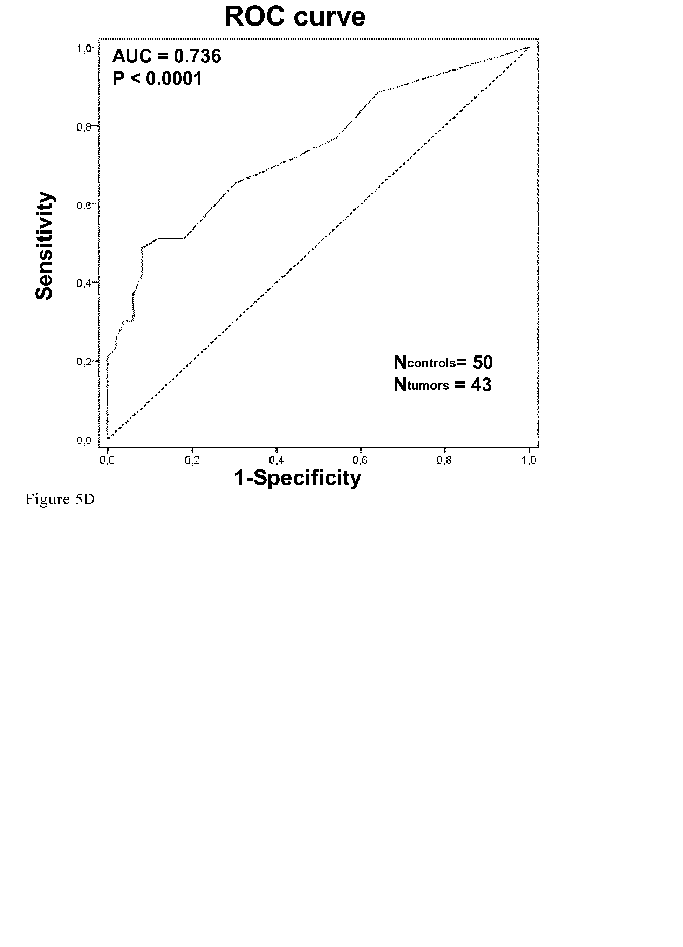

Figure 5: **A)** Table illustrating the median methylation values of a three panel locus (*SPAG6, PER1,* and *NKX2-6)* of breast cancer (DCIS) associated serum samples compared to control samples. As shown the methylation level allows to determine sensitivity and specificity of the analysis **B)** ROC curve analysis of the three panel biomarker performance is shown. A cut-off value of 1.6% methylation was defined for positive detection of disease; the specificity of the panel is still 94% with a sensitivity of 38%. **C)** Table illustrating the median methylation values of a three panel locus of breast cancer (invasive tumor) associated serum samples compared to control samples. As shown the methylation level allows to determine sensitivity and specificity of the analysis **D)** ROC curve analysis of the three panel biomarker performance is shown. A cut-off value of 1.6% methylation was defined for positive detection of disease; the specificity of the panel is still 94% with a sensitivity of 37%.

### Detailed description of the present invention

In a first aspect, the present invention provides a method for, preferably early, diagnosis of breast cancer or pre-forms thereof in a subject, comprising a) determining the level or amount of methylation of the promoter of the *SPAG6-* and/or *PER1-* and/or *NKX2-6*-gene, in particular, of the nucleic acid sequences of SEQ ID No. 1 and/or SEQ ID No. 2 and/or SEQ ID No. 3, in a sample of said subject; and b) diagnosing or identifying breast cancer or pre-forms thereof based on the level or amount of methylation of the promoter of the *SPAG6-* and/or *PER1-* and/or *NKX2-6-*gene.

That is, the present inventors recognized the depending on the level or amount of methylation of the promoter of the *SPAG6-* and/or *PER1-* and/or *NKX2-6*-gene, in a body fluid sample of a sample suspected to suffer from breast cancer or pre-forms thereof, or being at risk of developing breast cancer or pre-forms thereof, it is possible to diagnose or identify breast cancer or pre-forms thereof in said subject, preferably based on an elevated methylation level or amount of said biomarker relative to a reference value.

It has been recognized that elevated level or amounts of methylation of the promoter of *SPAG6-* and/or *PER1-* and/or *NKX2-6*-gene, in particular, of a nucleic acid sequence is of SEQ ID No. 1 and/or of SEQ ID No. 2 and/or SEQ ID No. 3 in a sample of the subject suspected to be affected from breast cancer or having a risk of developing breast cancer allows to diagnose for or identify the same, hence, determining the level or amount of methylation of said promoters represents a promising biomarker allowing to diagnose or identify said disease, determining disease's severity and response to treatment as well as allowing for monitoring the progression of therapy as well as a relapse of cancer and the stratification of therapeutic regimen of a patient suffering from breast cancer or pre-forms thereof. In this connection, diagnosis may include the determination of the stage and grade of breast cancer, respectively.

SPAG6: Sperm-associated antigen 6 (Spag6) is a Chlamydomonas reinhardtii PF16 homologous gene detected in the human testis and is crucial for sperm motility. Neuronal migration is a dynamic process requiring coordinated cytoskeletal remodeling, and Spag6 is co-localized with microtubules.

NKX2-6: NKX2.6 gene encodes a homeodomain transcription factor crucial for cardiovascular development.

PER1 is the gene circadian clock gene Period 1 (PER1).

By "gene" is meant not only the particular sequences found in the publicly available database entries, but also encompasses transcript and nucleotide variants of these sequences. The term may also encompass any gene which is taken from the family to which the named "gene" belongs with the proviso that methylation or another epigenetic modification of the "gene" is linked to breast cancer.

In the context of the present invention, the term "body fluid sample" or "sample of the body fluid" is a biological sample isolated from the subject which can include without being limited thereto, whole blood, serum and plasma as well as urine and nipple aspirate. Preferably, the body fluid is serum or plasma. However, samples useful in the methods according to the present invention for diagnostic, prognostic, or personalised medicinal uses can be obtained also from surgical samples, such as biopsies or fine needle aspirates, from paraffin embedded tissues, from frozen tumor tissue samples, or from fresh tumor tissue samples, from a fresh or frozen body fluid being present in liquid form..

A "subject" in the context of the present invention is preferably a mammal. The mammal can be a human, non-human primate, mouse, rat, dog, cat, horse or cow but are not limited to these examples. A subject can be male or female. A subject can be one who has been previously diagnosed with or identified as suffering from or having breast cancer and, optionally, but need not have already undergone treatment for said disease or disorder. A subject can also be one who has been diagnosed with or identified as suffering from breast cancer, but show improvements in the disease as a result of receiving one or more treatments for said disease or disorder. Moreover, a subject may also be one who has not been briefly diagnosed or identified as suffering from breast cancer. A subject can also be one who is suffering from or at risk of developing breast cancer, e.g., due to genetic predisposition.

The term "determining" as used herein refers to assessing the presence, absence, quantity, level or amount of either a given substance within a clinical or subject derived sample. In particular, the term "determining" refers to assess physically the level or amount of the methylation of a given DNA sequence.

The term "breast cancer" refers to a cancer that develops from breast tissue. The cancer may comprise all types of breast cancer as classified by several grading systems. The classification includes histopathology data, grade of the breast cancer as well as stages thereof. For example, breast cancer classification divides breast cancer into categories according to different schemes, each based on different criteria and serving a different purpose.

In the present case, the breast cancer particularly includes early stages and pre-cancerous stage, including the ductal carcinoma in situ (DCIS), also known as intra-ductal carcinoma, a form representing a pre-cancerous or non-invasive cancerous lesion of the breast. DCIS is classified as stage 0. This stage eventually develops with a high rate into advanced breast cancer types. DCIS is the most common type of pre-cancer in women. As used herein, DCIS is regarded as a breast cancer accordingly.

In addition, the early stage of breast cancer defined as early invasive tumours (pT1, pN0) is included. Suitable stages of breast cancer to which the invention is applicable are listed in the tables in the experimental section herein.

As used herein, the term "comprise" or "comprising" as well as the terms "contain" or "containing" include the embodiments of "consist of" or "consisting of".

In a further aspect, the present invention relates to a method for monitoring the development, progress or relapse of breast cancer or pre-forms thereof in a subject afflicted with, suspected to be afflicted with or having a risk of developing breast cancer or pre-forms thereof, comprising
a) determining the level or amount of methylation of the promoter of the *SPAG6*and/or *PER1-* and/or *NKX2-6*-gene, in particular, of the nucleic acid sequences of SEQ ID No. 1 and/or SEQ ID No. 2 and/or SEQ ID No. 3, at a first time point and, optionally,
b) determining the level or amount of methylation of the promoter of the *SPAG6-* and/or *PER1-* and/or *NKX2-6*-gene, in particular, of the nucleic acid sequences of SEQ ID No. 1 and/or SEQ ID No. 2 and/or SEQ ID No. 3, at a second time point; and optionally,
c) comparing the level or amount of methylation determined in step a) to the level or amount detected in step b) or to a reference value.

In this connection, an increase of the level or amount of methylation is indicative for the presence, a progression or worsening of the disease.

In another embodiment, the present invention relates to a method for the stratification of the therapeutic regimen of a subject suspected to be afflicted with breast cancer or being at risk of developing breast cancer or pre-forms, optionally in combination with mammography-based breast cancer detection and/or MRI-based breast cancer detection, comprising
a) determining the level or amount of methylation of the promoter of the *SPAG6-* and/or *PER1-* and/or *NKX2-6*-gene, in particular, of the nucleic acid sequences of SEQ ID No. 1 and/or SEQ ID No. 2 and/or SEQ ID No. 3; and
b) determining the therapeutic regimen of said subject based on the level or amount of methylation of the promoter of *SPAG6-* and/or *PER1-* and/or *NKX2-6-*gene, optionally further on the basis of the results of mammography-based breast cancer recognition and/or MRI-based breast cancer detection.

Not to be bound to theory, it is submitted that depending on the level or amount of methylation, the stage of breast cancer can be determined, and, thus, the artisan is able to select an appropriate therapeutic regimen from the regimens known in the art.

In an embodiment, the breast cancer is a breast cancer of a non-invasive ductal carcinoma in situ (DCIS) or of early invasive tumour (pT1, pN0).

That is, in an embodiment, the methods according to the present invention are useful for early diagnosis or determining the risk of development of breast cancer or pre-forms thereof, as described herein.

It has been recognized that a high level or amount of methylation is regarded as an indicator of being afflicted with breast carcinoma or pre-forms thereof or being at risk of developing breast cancer or pre-forms thereof or for the risk of development or of relapse of breast cancer, respectively.

As used herein, the term "pre-forms thereof" refers to pre-cancerous or premalignant tissues or cells having disease-relevant changes in their genetic and epigenetic genome configuration that support disease progression over time.

The sample from the subject may be a sample or body fluid or body tissue of said patient. Typically, the sample is in a liquid from. In an embodiment, the sample is a blood sample, like whole blood, serum or plasma, alternatively, the sample is urine or nipple aspirate. The sample typically contains cfDNA in case of body fluid and, in addition, is typically a cell free liquid sample,

Further, where a first sample is taken from the subject, said first sample may be taken from a subject prior to the treatment for breast cancer or pre-forms thereof and/or the second sample is taken from the subject after being treated for breast cancer or pre-forms thereof accordingly.

The method according to the present invention may be conducted in concert with imaging techniques including mammography and MRI.

Determination of the methylation status may be achieved through any suitable means. Suitable examples include bisulphite genomic sequencing and/or by methylation specific PCR. Various techniques for assessing methylation status are known in the art and can be used in conjunction with the present invention: sequencing, methylation-specific PCR (MS-PCR), melting curve methylation-specific PCR(McMS-PCR), MLPA with or without bisulphite treatment, QAMA, MSRE-PCR, MethyLight- or ConLight-MSP, bisulphite conversion-specific methylation-specific PCR (BS-MSP), COBRA (which relies upon use of restriction enzymes to reveal methylation dependent sequence differences in PCR products of sodium bisulphite - treated DNA), methylation-sensitive single-nucleotide primer extension conformation(MS-SNuPE), methylation-sensitive single-strand conformation analysis (MS-SSCA), Melting curve combined bisulphite restriction analysis, PyroMethA, HeavyMethyl, MALDI-TOF, MassARRAY, Quantitative analysis of methylated alleles (QAMA), enzymatic regional methylation assay (ERMA), QBSUPT, MethylQuant, Quantitative PCR sequencing and oligonucleotide-based microarray systems, Pyrosequencing, Next Generation Sequencing, Meth-DOP-PCR. A review of some useful techniques for DNA methylation analysis is provided e.g. in Nature Reviews, 2003, Vol.3, 253-266, which references are incorporated herein in their entirety. Techniques for assessing methylation status are based on distinct approaches. Some include use of endonucleases. Such endonucleases may either preferentially cleave methylated recognition sites relative to non-methylated recognition sites or preferentially cleave non-methylated relative to methylated recognition sites. Differences in cleavage pattern are indicative for the presence or absence of a methylated CpG dinucleotide. Cleavage patterns can be detected directly, or after a further reaction which creates products which are easily distinguishable. Means which detect altered size and/or charge can be used to detect modified products, including but not limited to electrophoresis, chromatography, and mass spectrometry.

Alternatively, the identification of methylated CpG dinucleotides may utilize the ability of the methyl binding domain (MBD) of the MeCP2 protein to selectively bind to methylated DNA sequences. The MBD may also be obtained from MBP, MBP2, MBP4, poly-MBD or from reagents such as antibodies binding to methylated nucleic acid. The MBD may be immobilized to a solid matrix and used for preparative column chromatography to isolate highly methylated DNA sequences. Variant forms such as expressed His-tagged methyl-CpG binding domain may be used to selectively bind to methylated DNA sequences. Eventually, restriction endonuclease digested genomic DNA is contacted with expressed His-tagged methyl-CpG binding domain. Other methods are well known in the art and include amongst others methylated-CpG island recovery assay (MIRA). Another method, MB-PCR, uses a recombinant, bivalent methyl-CpG-binding polypeptide immobilized on the walls of a PCR vessel to capture methylated DNA and the subsequent detection of bound methylated DNA by PCR.

Further approaches for detecting methylated CpG dinucleotide motifs use chemical reagents that selectively modify either the methylated or non-methylated form of CpG dinucleotide motifs. Suitable chemical reagents include hydrazine and bisulphite ions. The methods of the invention may use bisulphite ions, in certain embodiments. The bisulphite conversion relies on treatment of DNA samples with sodium bisulphite which converts unmethylated cytosine to uracil, while methylated cytosines are maintained. This conversion finally results in a change in the sequence of the original DNA. It is general knowledge that the resulting uracil has the base pairing behaviour of thymidine which differs from cytosine base pairing behaviour. This makes the discrimination between methylated and non-methylated cytosines possible. Useful conventional techniques of molecular biology and nucleic acid chemistry for assessing sequence differences are well known in the art and explained in textbooks.

Some techniques use primers for assessing the methylation status at CpG dinucleotides. Two approaches to primer design are possible. Firstly, primers may be designed that themselves do not cover any potential sites of DNA methylation. Sequence variations at sites of differential methylation are located between the two primers and visualisation of the sequence variation requires further assay steps. Such primers are used in bisulphite genomic sequencing, COBRA, Ms-SnuPE and several other techniques. Secondly, primers may be designed that hybridize specifically with either the methylated or unmethylated version of the initial treated sequence. After hybridization, an amplification reaction can be performed and amplification products assayed using any detection system known in the art. The presence of an amplification product indicates that a sample hybridized to the primer. The specificity of the primer indicates whether the DNA had been modified or not, which in turn indicates whether the DNA had been methylated or not. If there is a sufficient region of complementarity, e.g., 12, 15, 18, or 20 nucleotides, to the target, then the primer may also contain additional nucleotide residues that do not interfere with hybridization but may be useful for other manipulations. Examples of such other residues may be sites for restriction endonuclease cleavage, for ligand binding or for factor binding or linkers or repeats. The oligonucleotide primers may or may not be such that they are specific for modified methylated residues.

A further way to distinguish between modified and unmodified nucleic acid is to use oligonucleotide probes. Such probes may hybridize directly to modified nucleic acid or to further products of modified nucleic acid, such as products obtained by amplification. Probe-based assays exploit the oligonucleotide hybridisation to specific sequences and subsequent detection of the hybrid. There may also be further purification steps before the amplification product is detected e.g. a precipitation step. Oligonucleotide probes may be labelled using any detection system known in the art. These include but are not limited to fluorescent moieties, radioisotope labelled moieties, bioluminescent moieties, luminescent moieties, chemiluminescent moieties, enzymes, substrates, receptors, or ligands.

In the MSP approach, DNA may be amplified using primer pairs designed to distinguish methylated from unmethylated DNA by taking advantage of sequence differences as a result of sodium-bisulphite treatment (WO 97/46705). For example, bisulphite ions modify non-methylated cytosine bases, changing them to uracil bases. Uracil bases hybridize to adenine bases under hybridization conditions. Thus an oligonucleotide primer which comprises adenine bases in place of guanine bases would hybridize to the bisulphite-modified DNA, whereas an oligonucleotide primer containing the guanine bases would hybridize to the non-modified (methylated) cytosine residues in the DNA. Amplification using a DNA polymerase and a second primer yield amplification products which can be readily observed, which in turn indicates whether the DNA had been methylated or not. Whereas PCR is a preferred amplification method, variants on this basic technique such as nested PCR and multiplex PCR are also included within the scope of the invention.

That is, the method according to the present invention relates to determining methylation of the promoter of *SPAG6-* and/or *PER1-* and/or *NKX2-6*-gene comprising determining methylation of single CpGs.

That is, the sample, in particular, the blood sample, like the serum sample, is obtained and DNA is isolated thereof. The level or amount of methylation is preferably determined based on the bisulfite method. That is, the DNA is treated with bisulfite before determining its pattern of methylation. In animals, methylation is predominately in the addition of a methyl group to the carbon 5-postion of cytosine residues of the dinucleotide CpG, and it is implicated in repression of transcriptional activity. In the human DNA, typically 3% of cytosine (C) is methylated. Further, most cytosines are methylated in CpG dinucleotides. Methylation is involved in silencing gene expression.

Treatment of DNA with bisulfite converts cytosine residues to uracil but leaves 5'-methyl cytosine residues unaffected. Thus, by bisulfite treatment specific changes in the DNA sequence depending on the methylation status of individual cytosine residues can be introduced. Due to the individual changes in cytosine residues, it is possible to obtain single nucleotide resolution information about the methylation status of a sequence of DNA.

The converted DNA, i.e. the DNA obtained after bisulfite treatment, is then analysed for the presence of methylated cytosine. The analysis can be based on sequencing methods or on PCR-based methods or combinations thereof. Basically, it is possible to allocate the approaches for analysis into two classes, namely the non-methylation specific PCR-based methods and the methylation specific PCR, also called MSP.

That is, it is possible determining the level or amount of methylation according to the present invention by different methods including the following: Direct sequencing, pyrosequencing, methylation sensitive single strand confirmation analysis, high resolution melting analysis, methylation sensitive single nucleotide primer extension or base specific cleavage using MALDI-TOF analysis.

Further, methylation specific PCR (MSP) may be applied, in particular, quantitative MSP analysis. Moreover, micro arraying based methods may be used.

It is desired that the determination is based on pyro-sequencing or qMSP (quantitative methylation specific PCR). The skilled person is well aware of said methods and conducting the same for determining the level or amount of methylation of the specified genes.

A specific example of the MSP technique is designated real-time quantitative MSP (qMSP), and permits reliable quantification of methylated DNA in real time or at end point. Real-time methods are generally based on the continuous optical monitoring of an amplification procedure and utilise fluorescently labelled reagents whose incorporation in a product can be quantified and whose quantification is indicative of copy number of that sequence in the template. One such reagent is a fluorescent dye, called SYBR Green I that preferentially binds double-stranded DNA and whose fluorescence is greatly enhanced by binding of double-stranded DNA. Alternatively, labelled primers and/or labelled probes can be used for quantification. They represent a specific application of the well known and commercially available real-time amplification techniques such as TAQMAN(R), MOLECULAR BEACONS(R), AMPLIFLUOR(R) and SCORPION(R), DzyNA(R), PlexorTM etc. In the real-time PCR systems, it is possible to monitor the PCR reaction during the exponential phase where the first significant increase in the amount of PCR product correlates to the initial amount of target template.

For pyrosequencing, suitable primer pairs including probes are Seq. ID Nos. 6 to 8 for the SPAG6 gene, Seq. ID Nos. 9 to 11 for the PER1 gene, and Seq. ID Nos.12 to 14, respectively for the NKX2-6 gene.

In an embodiment, at least two out of three *SPAG6-, PER1-* and *NKX2-6-*genes are analysed (two marker panel) including the combination of *SPAG6* and *PER1, SPAG6* and *NKX2-6,* and *PER1* and *NKX2-6.* In an embodiment, all of the three promoter regions of the *SPAG6-, PER1-* and *NKX2-6*-genes are analysed (three marker panel).

In another embodiment, the method includes also the determination of the level or amount of methylation of the promoter of at least one of *DKK3-* and *ITlH5*-gene in particular, of the nucleic acid sequence of SEQ ID No. 4 and/or SEQ ID No. 5, in the sample of the subject. Suitable primer sets for pyro-sequencing of IHIT5-gene are Seq. ID Nos.15 to 17 and Seq. ID Nos.18 to 20, respectively.

In the context of the present invention, the term "reference value" refers to an index value, or a value derived from one or more breast cancer risk prediction algorithms or computed indices, a value derived from a subject with the same disease or disorder, or a value derived from the subject diagnosed with or identified as suffering from breast cancer. In particular, e.g. in case of the method for diagnosing or identifying breast cancer or pre-forms thereof, the reference value is obtained from subjects not afflicted with breast cancer or pre-forms thereof and, in addition, the reference value represents a range or index obtained from at least two samples collected from subjects not afflicted with breast cancer or pre-forms thereof.

The increase in the level or amount of methylation of the promoter or of *SPAG6-, PER1-* and *NKX2-6*-gene, optionally, the *DKK3-* and/or *ITIH5*-gene, is for example at least 10 %, at least 15 %, at least 20 %, at least 25 %, or at least 50 % of the reference value or normal control level, preferably, the increase is at least 100 %. Embodiments and methods are applied allowing determining the methylation of single CpG present in the promoter of the genes of the present invention. For example, by applying pyrosequencing or MSP, in particular, pyrosequencing or qMSP, it is possible to determining the level or amount of methylation of single CpG dinucleotides present in the promoter regions, accordingly.

The promoter region of the *SPAG6*-gene comprises 10 CpG dinucleotides, the *PER1* promoter comprises two CpGs and the *NKX2-6* promoter comprises four CpG. The CpG are shown in tables 1 to 3 below.

**Table 1: CpG Dinucleotides SPAG6 promoter**

| | ***SPAG6* gene** |
|---|---|
| | **Nucleotide position (5'-3')^{a}** |
| **CpG Number** | |
| **1** | 120-121 |
| **2** | 123-124 |
| **3** | 125-126 |
| **4** | 127-128 |
| **5** | 135-136 |
| **6** | 138-139 |
| **7** | 144-145 |
| **8** | 146-147 |
| **9** | 155-156 |
| **10** | 157-158 |

| | |
|---|---|
| **^{a}Position is related to the *SPAG6* gene of Seq. ID No. 1 (5'-3')** | |

**Table 2: CpG Dinucleotides PER1 promoter**

| | ***PER1* gene** |
|---|---|
| | **Nucleotide position (5'-3')^{a}** |
| **CpG Number** | |
| **1** | 327-328 |
| **2** \| | 336-337 |

| | |
|---|---|
| **^{a}Position is related to the *PER1* gene of Seq. ID No. 2 (5'-3')** | |

**Table 3: CpG Dinucleotides NKX2-6 promoter**

| | ***NKX2-6* gene** |
|---|---|
| | **Nucleotide position (5'-3')^{a}** |
| **CpG Number** | |
| **1** | 201-202 |
| **2** | 209-210 |
| **3** | 211-212 |
| **4** | 231-232 |

| | |
|---|---|
| **^{a}Position is related to the *NKX2-6* gene of Seq. ID No. 3 (5'-3')** | |

In table 4 CpG dinucleotides for the IHIT5-promoter are shown:

**Table 4:**

| | **ITIH5 gene** |
|---|---|
| | **Nucleotide position (5'-3')^{a}** |
| **CpG Number** | |
| **1** | 487-488 |
| **2** | 489-490 |
| **3** | 499-500 |
| **4** | 519-520 |
| **5** | 664-665 |
| **6** | 692-693 |
| **7** | 723-724 |
| **8** | 725-726 |
| **9** | 728-729 |

| | |
|---|---|
| **^{a}Position is related to the ITIH5 gene Seq. ID No. 5 (5'-3')** | |

For example, suitable CpG include at least one of CpG 2 and 9 of the *SPAG6* promoter and/or the CpG 2 of the *PER1* promoter and/or the CpG 2 and/or 4 of the *NKX2-6* promoter according to tables 1 to 3. Hence, an embodiment of the present invention relates to the determination of the methylation of at least one of CpG 2 and 9 of *SPAG6* promoter and/or the CpG2 of the *PER1* promoter and/or the CPG 2 and/or 4 of the *NKX2-6* promoter gene according to tables 1 to 3, respectively.

It is preferred that the methylation of single or individually combined CpG sites is determined by pyro-sequencing or qMSP.

It is preferred that the level of a methylation of the CpGs is twofold, like threefold or more above the normal value.

It is particular preferred that the level of methylation is at least threefold, like fourfold higher in subjects afflicted or deemed to be afflicted with breast cancer compared to a subject not afflicted.

Suitable controls may need to be incorporated in order to ensure the method chosen is working correctly and reliably. Suitable controls may include assessing the methylation status of a gene known to be methylated. This experiment acts as a positive control to help to ensure that false negative results are not obtained. The gene may be one which is known to be methylated in the sample under investigation or it may have been artificially methylated, for example by using a suitable methyltransferase enzyme, such as Sssl methyltransferase. In one embodiment, the gene selected from *SPAG6-, PER1-* and *NKX2-6-*gene, may be assessed in normal (i.e. non-cancerous breast) cells, following treatment with Sssl methyltransferase, as a positive control.

Additionally or alternatively, suitable negative controls may be employed with the methods of the invention. Here, suitable controls may include assessing the methylation status of a gene known to be unmethylated or a gene that has been artificially demethylated. This experiment acts as a negative control to ensure that false positive results are not obtained. In one embodiment, the gene selected from *SPAG6-, PER1-* and *NKX2-6*-gene may be assessed in normal (breast) cells as a negative control, since it has been shown for the first time herein that these genes are unmethylated in normal (breast) tissues.

All methods of the present invention may be used in connection with breast cancer. To attain high rates of tumor detection, it may be advantageous to complement the methods of the invention with established methods for breast cancer identification. Non-invasive methods may be especially suitable for use in combination with the non-invasive methods of the invention.

For example, the method may be combined with known methods for diagnosing or analysing breast cancer or pre-forms thereof, e.g. MRI or mammography. That is, in an embodiment, the method according to the present invention complements the method of the established methods for breast cancer identification, in particular, mammography. In an embodiment, in a first step the method of determining the amount or level of methylation according to the present invention is conducted followed by mammography and/or MRI.

It is preferred that the analysis of the values obtained after determining the amount or level of methylation is analysed by ROC (receiver operating characteristic). Based on said ROC analysis, it is possible to identify a cut off level of sensitivity above 50 or even above 60% while having a specificity of above 90%, e.g. of having 95% specificity. That is, it is possible to have 95% specificity while sensitivity is above 40% based on ROC analysis with early breast cancer. This is particularly true for the preferred embodiment of the analysis of all three of the promoter of the *SPAG6-, PER1-* and *NKX2-6*-gene.

It is noteworthy that for the methods according to the present invention only small amounts of the sample are required and, in addition, that the methods do not necessitate any surgery steps when using body fluid, like blood as biological sample. In addition, it is possible to define cut off levels, thus, providing suitable methods for diagnosis and stratification as well as determining the treatment course of breast cancer. Hence, the methods according to the present invention are particularly valuable for medical prevention of cancer as well as for risk assessment of in subjects suffering from breast cancer including repalse of the tumor. Moreover, the present method is comfortable for the subject examined.

In a further aspect, the present invention relates to the use of at least one promoter of the *SPAG6*-gene or the promoter of the *PER1*-gene or the promoter of the *NKX2-6*-gene, in particular, level or amount of methylation thereof, as a biomarker for breast cancer or brief forms thereof. In particular, the present invention provides the use of the three marker panel of the promoter of the *SPAG6-* and/or *PER1-* and/or *NKX2-6*-gene, in particular, of the nucleic acid sequences of SEQ ID No. 1 and/or SEQ ID No. 2 and/or SEQ ID No. 3.

Finally a kit is provided for detecting a pre-disposition to, or the incidence of a patient to breast cancer in a sample (the sample comprising nucleic acid molecules from breast cells) comprising at least one primer pair and/or probe for determining the methylation status of each gene in a panel of genes when a panel of genes comprise, consist essentially of or consist of a panel of genes selected from *SPAG6-* and/or *PER1-* and/or *NKX2-6*-gene. Optionally, *DKK3* and/or *ITIH5*-gene may be analysed.

As discussed herein, these genes have been shown to be useful in predicting or diagnosing breast cancer, non-invasively, with excellent sensitivity and specificity. Suitable primer pairs for determining the methylation status of each of the genes of the panel are set forth in the examples below. The primers and/or probe may permit direct determination of the methylation status of the panel of genes, for example following bisulphite treatment of the DNA. Thus, they may be MSP or bisulphite sequencing primers for example. The kits may additionally include one or more probes for real-time or end-point detection. The probes may additionally or alternatively permit direct determination of the methylation status of the panel of genes, for example following bisulphite treatment of the DNA. Blocking probes may also be utilised in certain embodiments, according to the Heavymethyl technique.

The primers and/or probe may investigate the methylation status of the relevant gene or genes. In certain embodiments, the primers and/or probe may investigate the methylation status within, or between, and optionally including, the primer and/or probe binding sites of the primers and/or probes listed in the examples. In specific embodiments, the primers and/or probes may investigate the methylation status, within or between the genomic locations identified in the examples and in figures. Thus, for example, the primers and/or probes may investigate the genomic region between (and including) nucleotide 123-124 and nucleotide 155-156 for *SPAG6* according to Seq. ID No.1 and/or the genomic region between (and including) nucleotide 336 and nucleotide 337 for *PER1* according to Seq. ID No.2 and/or the genomic region between (and including) nucleotide 209-210 and nucleotide 231-232 for *NKX2-6* according to Seq. ID No.3. Preferably, the primer and probes are primer pairs and corresponding probes as defined above.

The kit comprises means for determining the level or amount of methylation of the promoter of the *SPAG6-* and/or *PER1-* and/or *NKX2-6*-gene, optionally, means for determining the level or amount of methylation of the promoter of the DKK3-gene and/or *ITIH5*-gene*,* in particular, of the nucleic acid sequence of SEQ ID No. 1 and/or SEQ ID No. 2 and/or SEQ ID No. 3 and/or SEQ ID No. 4 and/or SEQ ID No. 5, in a body fluid sample of a subject to be tested, in particular, a serum sample, and instructions on how to use said test kit for a method according to the present invention for the stratification of the therapeutic regimen, for diagnosing or identifying or for monitoring the progression of breast cancer or pre-forms in a subject supposed to have or being afflicted with breast cancer or pre-forms.

The kit may enable the test to be carried out in a single reaction, for example, including soluble labelled primers or probes or by selecting amplification products which can be readily distinguished according to size, molecular weight etc.

The kit may be for use in MSP or pyrosequencing and may enable a real time detection version of MSP. In some embodiments, the kit permits an end-point detection version of MSP to be carried out. Thus, the means for detecting an epigenetic change may comprise, consist substantially of or consist of suitable primers for determining whether at least one gene selected from the promoter of *SPAG6-* and/or *PER1-* and/or *NKX2-6*-gene (alone or together, optionally, with additional genes) is methylated.

These primers may comprise any of the primers discussed in detail in respect of the various methods of the invention which may be employed in order to determine the methylation status of the relevant (at least one) gene, and variants thereof.

The kit may further comprise probes for real-time detection of amplification products. The probes may comprise any suitable probe type for real-time detection; non-limiting examples include use of TAQMAN probes and/or MOLECULAR BEACONS probes and/or AMPLIFLUOR primers and/or FRET probes and/or SCORPION primers and/or oligonucleotide blockers. Such kits for real-time detection may also be used for end-point detection.

The primers and/or probes may permit direct determination of the methylation status of the at least one gene in all of the kits of the invention, for example following bisulphite treatment of the (DNA in the) sample, as discussed herein.

The primers and/or probes may be labelled as required. FAM and DABCYL are representative examples of fluorescent markers which can participate in FRET to provide a reliable indicator of amplification, as discussed herein. Other fluorophores and quenchers may be employed, in particular as FRET pairs, as desired and as would be appreciated by a skilled person.

The primers and/or probe may investigate the methylation status, of the relevant gene or genes. In certain embodiments, the primers and/or probe may investigate the methylation status within, or between, and optionally including, the primer and/or probe binding sites of the primers and/or probes listed in the table.

As indicated herein above, the kit may comprise means for processing a blood sample. Such means for processing a blood sample may comprise a stabilising buffer in certain embodiments. Suitable stabilising buffers are described herein and may incorporate appropriate mixtures of buffering and osmolarity adjustment ingredients. Examples include STABILUR tablets, available from Cargille Labs and preservative tubes available from CellSave (CellSave Preservative Tubes).

The kit may further incorporate reagents for extraction / isolation / concentration/purification of DNA in certain embodiments. In further embodiments, the kit may also incorporate a sealable vessel for collection of a blood sample. In certain embodiments, the kit of the invention further comprises a reagent which modifies unmethylated cytosine (but not methylated cytosine) or vice versa in detectable fashion. This allows methylated residues to be distinguished from non-methylated residues. In certain embodiments, the reagent converts unmethylated cytosine residues to a different nucleotide (uracil) but methylated residues are not converted. In certain embodiments, the reagent comprises bisulphite, preferably sodium bisulphite but may comprise hydrazine for example.

As discussed, suitable controls may be utilised in order to act as quality control for the methods and be included in the kit of the invention. One example of a suitable internal reference gene, which is generally unmethylated, but may be treated so as to be methylated, is [beta]-actin. The kit of the invention may further comprise primers for the amplification of a control nucleic acid which may comprise at least one gene selected from *SPAG6-, PER1-* and *NKX2-6*-gene in unmethylated and/or methylated form.

The kits of the invention may additionally include suitable buffers and other reagents for carrying out the claimed methods of the invention. In certain embodiments, the kit of the invention further comprises, consists essentially of, or consists of nucleic acid amplification buffers.

The kit may also additionally comprise, consist essentially of or consist of enzymes to catalyze nucleic acid amplification. Thus, the kit may also additionally comprise, consist essentially of or consist of a suitable polymerase for nucleic acid amplification. Examples include those from both family A and family B type polymerases, such as Taq, Pfu, Vent etc.

The various components of the kit may be packaged separately in separate compartments or may, for example be stored together where appropriate. The kit may also incorporate suitable instructions for use, which may be printed on a separate sheet or incorporated into the kit packaging for example.

That is, in another aspect the present invention relates to kit for use in a method according to the present invention for predicting the clinical outcome or determining the treatment course in a subject, for diagnosing or identifying breast cancer in a subject, for the stratification of the therapeutic regimen, or for monitoring the progression of breast cancer in a subject supposed to have or afflicted with breast cancer, said kit comprises a means for determining the level or amount of methylation of the promoter of the *SPAG6-, PER1-* and/or *NKX2-6-*gene, in particular, of the nucleic acid sequence of SEQ ID No. 1 and/or SEQ ID No. 2 and/or SEQ ID No. 3 in a body fluid sample of a subject to be tested, in particular, of a serum sample, and instructions on how to use said test kit for a method according to the present invention.

The kits according to the present invention are adapted to or designed for use in a method according to the present invention.

It is particularly preferred that said kit allows pyro-sequencing or qMSP of the biological sample, in particular, DNA obtained from a human sample of said subject.

Generally, it is preferred that in the method according to the present invention as well as in a kit according to the present invention all three promoters of the *SPAG6-, PER1-* and *NKX2-6*-gene, are analysed for methylation. Furthermore, it is preferred that the promoter of the *SPAG6-, PER1-* and *NKX2-6-*gene, in particular, the level or amount of methylation thereof, are used as a biomarker for breast cancer, optionally in combination with the promoter of the DKK3-gene and/or the promoter of the *ITIH5*-gene*.*

In another embodiment, the present invention relates to a method of treating breast cancers or pre-forms thereof applying biological or epigenetic therapies with biological or small molecule drugs. Depending on the pattern of marker gene promoters found to be methylated in the human biomaterial analyzed, a personalized treatment (precision medicine) may be selected. In the case of biological therapy the drug may be one or more of the tumor suppressor proteins or derivates thereof, that can be derived from the tumor suppressor genes (ITIH5, DKK3, SPAG1, PER1, NKX2-6) mentioned in this invention. In the case of epigenetic therapies the drug may be a DNA methyltransferase (DNMT) inhibitor or a histone deacetylase (HDAC) inhibitor. Further, the doctor can decide on the treatment either using cytostatika or surgery based on the status of methylation.

The above disclosure generally describes the present invention. A more complete understanding can be obtained by reference to the following specific examples of the embodiments of the invention without being limited thereto.

### Methods

### Patients and design study

Serum samples were obtained from the University Hospital Aachen. Only, serum samples from patients with primary breast cancer (pN0, pT1 cases) as well as patients with non-invasive DCIS were used. According to the relevant demographic data serum samples from healthy donors were age matched and used for controls. All patients gave informed consent for retention and analysis of their blood samples for research purposes, and the Ethics Committees of the respective centres approved the study.

### Serum sample preparation

The storage and processing conditions of blood samples were standardised: Blood samples (10 ml) from all study participants were obtained by venipuncture. Samples were immediately centrifuged at 2,000g for 10 minutes at room temperature, and 1 ml serum aliquots were stored at -80°C until use.

### CfDNA isolation from serum

CfDNA was extracted from 1 ml serum by using the QIAamp Circulating Nucleic Acid Kit (Qiagen, Hilden, Germany) according to the manufacturer's recommendations, eluted in 35 µl of TE buffer, and quantified spectrophotometrically.

### Bisulfite-modification

Bisulfite treatment of DNA was performed as previously described (Veeck J., et al., 2008, Oncogene, 27(6):865-76).

### Pyrosequencing

Based on the specific *SPAG6, PER1* and NKX2-6assays e.g. using the primer set according to Seq ID Nos. 6 to 8. 9 to 11, and 12 to 14, respectively, for determining methylation of CpG's shown in tables 1 2 and 3, respectively, pyrosequencing analysis was performed as previously described (Noetzel E., et al., 2010, Oncogene, 29(34):4814-25).

### Results

Initial DNA methylation analysis of the *SPAG6-, PER1-* and *NKX2-6*-gene promoter identified distinct regions with a high biomarker potential, respectively (Figures 1 to 3). Analysing *SPAG6, PER1* and NKX2-6 gene promoter methylation in serum samples of breast cancer patients, frequent methylation was detected achieving an overall-panel sensitivity (*SPAG6, PER1* and *NKX2-6)* of approximately 40% in non-invasive DCIS patients (table 5) and approximately 35% sensitivity in early invasive breast tumor patients (table 6). Biomarker performances of *SPAG6, PER1* and *NKX2-6* promoter regions were further optimized by using receiver operator characteristics (ROC) curve analysis. Of importance, this analysis provided CpG sites (named "best CpGs" in Figures 1 to 3) with best biomarker performance to discriminate between serum samples from breast cancer patients (DCIS and invasive tumours) and healthy individuals. Combining the DNA methylation biomarkers *SPAG6, PER1* and *NKX2-6,* an overall-three-gene panel sensitivity of 51 % (p<0.0001, AUC=0.810) with still 92% specificity and 49% sensitivity (p<0.0001, AUC=0.736) with still 92% specificity in DCIS (figure 4) and invasive breast cancer (figure 5), respectively, was obtained.

**Table 5: Non-invasive DCIS samples**

| | ***SPAG6* (CpG2/9)** | ***PER1* (CpG2)** | ***NKX2-6* (CpG2/4)** | ***SPAG6* + *NKX2-6* Panel** | ***SPAG6* + *NKX2-6* + *PER1* Panel** |
|---|---|---|---|---|---|
| **Cut**-**Off**-**Level^{a}** | ≥1.3 | ≥2.5 | ≥2.6 | ≥1.4 | ≥1.6 |
| **Sensitivity [%]** | 43.59 | 17.95 | 25.64 | 38.46 | 38.46 |
| **Specificity [%]** | 95 | 95 | 95 | 95 | 95 |
| **AUC^{b}** | 0.645 | 0.598 | 0.676 | 0.773 | 0.8108 |
| **P**-**value^{c}** | 0.019 | 0.114 | 0.005 | <0.001 | <0.001 |

| | | | | | |
|---|---|---|---|---|---|
| ^{a}Cut-Off-Level condition: 95% specificty. ^{b}AUC = Area Under Curve (ROC analysis-based). ^{c}Asymptotic significance level of 0.05 (95% AUC confidence intervals) | | | | | |

**Table 6: Invasive breast tumor samples**

| | ***SPAG6* (CpG2/9)** | ***PER1* (CpG2)** | ***NKX2-6* (CpG2/4)** | ***SPAG6* + *NKX2-6* Panel** | ***SPAG6* + *NKX2-6 + PER1* Panel** |
|---|---|---|---|---|---|
| **Cut**-**Off**-**Level^{a}** | ≥1.3 | ≥2.5 | ≥2.6 | ≥1.4 | ≥1.6 |
| **Sensitivity [%]** | 29.55 | 17.78 | 20.45 | 37.21 | 34.88 |
| **Specificity [%]** | 95 | 95 | 95 | 95 | 95 |
| **AUC^{b}** | 0.6627 | 0.638 | 0.591 | 0.679 | 0.736 |
| **P**-**value^{c}** | 0.007 | 0.020 | 0.128 | 0.003 | <0.001 |

| | | | | | |
|---|---|---|---|---|---|
| ^{a}Cut-Off-Level condition: 95% specificty. ^{b}AUC = Area Under Curve (ROC analysis-based). ^{c}Asymptotic significance level of 0.05 (95% AUC confidence intervals) | | | | | |

### Conclusion:

Three novel biomarker, i.e. *SPAG6, PER1* and *NKX2-6* gene promoter methylation, have been identified. Determine *SPAG6, PER1* and *NKX2-6* promoter methylation in serum (e.g. using pyrosequencing or qMSP) is suitable to detect primary breast cancer with very high sensitivity and specificity. Targeting the "best CpG" sites could improve the preferred 3-gene-panel biomarker performance, thus opens up a non-invasive alternative approach for early breast cancer diagnosis compared to current clinical standards as well as to recently published DNA methylation biomarkers candidates in breast cancer such as the *RASSF1A* gene.

## Claims

1. A method for diagnosis of breast cancer or pre-forms thereof in a subject, comprising
a) determining the level or amount of methylation of the promoter of the SPAG6- and/or PER1- and/or NKX2-6-gene, in particular, of the nucleic acid sequences of SEQ ID No. 1 and/or SEQ ID No. 2 and/or SEQ ID No. 3, in a sample of said subject; and
b) diagnosing or identifying breast cancer or pre-forms thereof based on the level or amount of methylation of the promoter of the SPAG6- and/or PER1- and/or NKX2-6-gene.

2. A method for monitoring the development, progress or relapse of breast cancer or pre-forms in a subject afflicted with, suspected to be afflicted with or having a risk of developing breast cancer, comprising
a) determining the level or amount of methylation of the promoter of the SPAG6- and/or PER1- and/or NKX2-6-gene, in particular, of the nucleic acid sequences of SEQ ID No. 1 and/or SEQ ID No. 2 and/or SEQ ID No. 3, at a first time point and, optionally,
b) determining the level or amount of methylation of the promoter of the SPAG6- and/or PER1- and/or NKX2-6-gene, in particular, of the nucleic acid sequences of SEQ ID No. 1 and/or SEQ ID No. 2 and/or SEQ ID No. 3, at a second time point; and optionally,
c) comparing the level or amount of methylation determined in step a) to the level or amount detected in step b) or to a reference value.

3. A method for the stratification of the therapeutic regimen of a subject suspected to be afflicted with breast cancer or being at risk of developing breast cancer or pre-forms thereof, optionally in combination with mammography-based breast cancer detection, comprising
a) determining the level or amount of methylation of the promoter of the SPAG6- and/or PER1- and/or NKX2-6-gene, in particular, of the nucleic acid sequences of SEQ ID No. 1 and/or SEQ ID No. 2 and/or SEQ ID No. 3; and
b) determining the therapeutic regimen of said subject based on the level or amount of methylation of the promoter of SPAG6- and/or PER1- and/or NKX2-6-gene, optionally further on the basis of the results of mammography-based breast cancer recognition.

4. The method according to any one of the preceding claims wherein the breast cancer is a breast cancer of non-invasive ductal carcinoma in situ (DCIS) or of early invasive tumour (pT1, pN0).

5. The method of any one of the preceding claims where a high level or amount of methylation is regarded as an indicator for being afflicted with breast cancer or pre-forms thereof or being at high risk of developing breast cancer or pre-forms thereof or for the risk of development of relapse of breast cancer, respectively.

6. The method according to any one of the preceding claims wherein the sample from the patient is a sample of body fluid or body tissue of said patient, in particular, of urine or blood, like whole blood or serum.

7. The method according to any one of claims 2 to 6 wherein the first sample is taking from the subject prior to the treatment for breast cancer or pre-forms thereof and/or the second sample is taken from a subject after being treated for breast cancer or pre-forms thereof.

8. The method to any one of the preceding claims wherein determining methylation of the promoter of the SPAG6- and/or PER1- and/or NKX2-6-gene comprises determining of methylation of single CpG.

9. The method according to claim 8 wherein determining the methylation of said promoter includes determining the level or amount of methylation of the CpGs 1 to 10 of the SPAG6 promoter and/or 1 and 2 of the PER1 promoter and of 1 to 4 of the NKX2-6 promoter according to tables 1 to 3 respectively.

10. The method according to any one of the preceding claims **characterized in that** the methylation is determined by pyrosequencing or qMSP.

11. A method according to any one of the preceding claims wherein at least the level of methylation of at least one of the CpG 2 and 9 of the SPAG6 promoter and/or the CpG 2 of the PER1 promoter and/or the CPG 2 and/or 6 of the NKX2-6 promoter according to table 1 to 3, respectively, is determined.

12. The method according to any one of the preceding claims wherein at least two out of the three SPAG6-, PER1- and NKX2-6-genes are analysed, in particular, wherein all three promoter regions of the SPAG6-, PER1- and NKX2-6-gene are analysed.

13. The method according to any one of the preceding claims further comprising determining the level or amount of methylation of the promoter of the DKK3- and/or ITIH5-gene, in particular, of the nucleic acid sequence of SEQ ID No. 4 and/or SEQ ID No. 5, in the sample of said subject.

14. A kit comprising means for determining the level or amount of methylation of the promoter of the SPAG6- and/or PER1- and/or NKX2-6-gene, optionally, means for determining the level or amount of methylation of the promoter of the DKK3-gene and/or ITIH5-gene, in particular, of the nucleic acid sequence of SEQ ID No. 1 and/or SEQ ID No. 2 and/or SEQ ID No. 3 and/or SEQ ID No. 4 and/or SEQ ID No. 5 in a body fluid sample of a subject to be tested, in particular, a serum sample, and instructions on how to use said test kit for a method according to any one of claims 1 to 13 for the stratification of the therapeutic regimen, for diagnosing or identifying or for monitoring the progression of breast cancer or pre-forms in a subject supposed to have or being afflicted with breast cancer or pre-forms.

15. The use of at least one of the promoter of the SPAG6-gene or the promoter of the PER1-gene or the promoter of the NKX2-6-gene, in particular, the level or amount of methylation thereof, as a biomarker for breast cancer or pre-forms thereof.
